# EUROPEAN PATENT APPLICATION

(11) **EP 4 537 896 A1**
(43) Date of publication of application: **16.04.2025**
(21) Application number: 23819936.8
(22) Date of filing: 09.06.2023
(51) Int. Cl.: A61N 5/06

(54) **METHOD FOR TREATING PARKINSON'S DISEASE THROUGH PHOTOSTIMULATION, AND DEVICE USED IN SAID METHOD**

(30) Priority: 10.06.2022 JP 2022094237
(71) Applicant: Juntendo Educational Foundation, Tokyo 113-8421 (JP); Tsubota Laboratory, Inc., Tokyo 160-0016 (JP)
(72) Inventor: HAYANO Motoshi, Tokyo 160-0016 (JP); TSUBOTA Kazuo, Tokyo 160-0016 (JP); HATTORI Nobutaka, Tokyo 113-8421 (JP); OYAMA Genko, Tokyo 113-8421 (JP)
(74) Representative: Plasseraud IP
(86) International application number: PCT/JP2023/021635
(87) International publication number: WO 2023/238956

(57) **Abstract**

The present invention provides a method of treating Parkinson's disease by light stimulation in which light of a specific wavelength such as violet light is irradiated at a specific blinking frequency. The present invention, by irradiating light of a specific wavelength such as violet light to a living body, carries out treatment and/or prophylaxis of Parkinson's disease by improving or suppressing at least one symptom selected from a group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis.

## Description

### Technical Field

The present invention relates to a method for treating a disease by light stimulation, and an apparatus used for the same, and more particularly, relates to a method for treating Parkinson's disease by light stimulation in which light of a specific wavelength such as violet light is irradiated at a specific blinking frequency, and an apparatus used for the same.

### Background Art

Effects of light on a human body have been studied from various perspectives in recent years, and reported on the basis of new findings. For instance, facts such as, circadian rhythm is improved by bathing in the sunlight (Non-Patent Document 1), light irradiated from a display such as a liquid-crystal display in which LED (light emitting diode) lighting and an LED is used as a backlight, have a substantial effect on body and mind (Non-Patent Document 2), and violet light prevents myopia and suppresses development of myopia (Patent Document 1) have been reported. Recently, in particular, inventors of the present invention have submitted an interesting report about effects of violet light on eyes, and in Patent Document 1 and Non-Patent Document 3 for example, it has been proposed that light of a specific wavelength is effective in preventing myopia and suppressing myopia, and with myopic population still on the rise globally in recent years, high expectations are placed.

Moreover, research and development of various therapeutic techniques has also been active, and research and development of therapeutic techniques with reduced burden on body, particularly by not using medication or reducing the use thereof, has been carried out.

For instance, In Patent Document 2 and Non-Patent Document 4, in research using mice suffering from Alzheimer disease, it has been reported that when tuning of gamma wave vibrations is recuperated in the brain, amyloid β protein accumulated in the brain is removed.

### Citation List

### Non-Patent Documents

Non-Patent Document 1 : Anti-aging Medicine by Megumi Hatori and Kazuo Tsubota, Japanese Society of Anti-aging Medicine Magazine Vol. 11, No. 3, 065 (385) - 072 (392), (2015)
Non-Patent Document 2: 'Blue Light Hazard' by Kazuo Tsubota, Published by Shueisha on November 20, 2013
Non-Patent Document 3: Hidemasa Torii et al., EBioMedicine, 'DOI: http://dx.doi.org/10.1016/j.ebiom.2016.12.007'
Non-Patent Document 4: NATURE, Vol. 540, 8, DECEMBER 2016, p. 231-235

### Patent Documents

Patent Document 1: WO2015/186723A1
Patent Document 2: US2017/0304584A1

### Summary of the Invention

### Problems to be Solved by the Invention

Parkinson's disease (PD) is a disease with the second largest number of patients after Alzheimer disease among neurodegenerative diseases, and is characterized by motor symptoms such as tremor and non-motor symptoms such as psychological symptoms. From a molecular and cellular biological point of view, a loss of substantia nigra dopaminergic neurons and formation of intracellular Lewy bodies made of α-synuclein and ubiquitin 1 has been acknowledged. Moreover, in a model mouse (Atg 7 knockout mice) of Parkinson's disease, deposition of p62 protein is observed. p62 is a protein having a ubiquitin binding region and an LC3 binding region, and has been known to have a function of carrying ubiquitinated protein to autophagosome. While research and development of a method for treating Parkinson's disease has been progressing actively, at the present stage, there is no method for fundamentally treating Parkinson's disease, and supportive measures which alleviate the symptoms is the main focus. The basis of the treatment is drug therapy, and a treatment for replenishing dopamine that is decreased is carried out. As a medication, medical agents such as dopamine precursor (levodopa) and dopamine agonist (for example, ropinirole) have been used. However, visual hallucination is a side effect of dopamine replacement therapy, and for improving the visual hallucination, reducing the dopamine replacement therapy and use of an antipsychotic medicine is necessary, and deterioration of motor symptoms becomes an issue.

The inventors of the present invention discovered that irradiation of light of a specific wavelength, particularly violet light (rays in a region of 360 nm to 400 nm), at a blinking frequency improves symptoms that are attributable to Parkinson's disease or the treatment thereof. An object of the present invention is to provide a method for treatment and/or prophylaxis of Parkinson's disease by irradiating light of a specific wavelength such as violet light constantly or at a specific blinking frequency, and an apparatus and a computer program used for the same.

The present invention includes providing a method for treatment and/or prophylaxis motor symptoms, non-motor symptoms and/or other elements of a patient suffering from Parkinson's disease that are attributable to Parkinson's disease or the treatment thereof, and an apparatus and a computer program used for treatment and/or prophylaxis thereof. Here, the symptoms that are attributable to the treatment of Parkinson's disease may be symptoms that are caused by dopamine replenishment therapy.

### Means for Solving the Problems

The present disclosure provides a method for treatment and/or prophylaxis of Parkinson's disease by irradiating light of a specific wavelength to a subject. More preferably, the present disclosure provides a method for treatment and/or prophylaxis of motor symptoms, non-motor symptoms and/or other elements of Parkinson's disease by irradiating violet light (VL) constantly or at a specific blinking frequency to the subject.

Moreover, according to one aspect, the present disclosure provides a method for treatment and/or prophylaxis of Parkinson's disease by irradiating light of a specific wavelength to a subject by controlling a light irradiating apparatus. In some embodiments, the light irradiating apparatus includes a light source which irradiates light of a specific wavelength constantly or at a specific blinking frequency to a living body, and a controller which controls the blinking frequency of the light source. The method includes a method for treatment and/or prophylaxis of motor symptoms, non-motor symptoms, and/or other elements in the subject by the light of a specific wavelength being irradiated to the subject by the light irradiating apparatus.

According to another aspect, the present disclosure is related to a method for treatment and/or prophylaxis of motor symptoms, non-motor symptoms, and/or other elements in a subject which requires treatment and/or prophylaxis, which includes irradiating light of a specific wavelength to the subject. The subject to which the method according to the present disclosure is applied may be a patient suffering from Parkinson's disease.

According to still another aspect, the present disclosure provides a method for treatment and/or prophylaxis of cognitive memory disorder, working memory disorder, dysphoria, anxiety, apathy, and psychological symptoms (hallucination, visual hallucination, and auditory hallucination) associated with Parkinsons's disease.

In other words, the present disclosure is as follows.

### [Item 1]

A method for treatment and/or prophylaxis of Parkinson's disease includes irradiating light of a specific wavelength to a subject by using a light irradiating apparatus.

### [Item 2]

The method according to item 1 which is a method for treatment and/or prophylaxis of Parkinson's disease, is characterized by treating and/or preventing motor symptoms and/or non-motor symptoms of Parkinson's disease.

### [Item 3]

The method according to item 2, wherein the motor symptom is at least one symptom selected from a group of symptoms consisting of difficulty in speaking, ptyalism (excessive drooling), difficulty in chewing, difficulty in swallowing, difficulty in feeding action, difficulty in changing clothes, difficulty in maintaining personal hygiene, difficulty in writing, decrease in hobbies and entertainment, difficulty in turning over, tremor, difficulty in getting up from bed, chair, and wheelchair, difficulty in walking, loss of balance, freezing of gait (FOG), stiffness in walking, difficulty in speech, loss of facial expression, rigidity, finger tapping, impairment in hand movement, impairment in pronation and supination movement of the hand, toe tapping, impairment in agility of the lower extremities, impairment in postural stability, difficulty in maintaining posture, general spontaneity of movement (bradykinesia), hand posture tremor, hand movement tremor, resting tremor amplitude, and restless leg syndrome.

### [Item 4]

The method according to one of items 2 and 3, wherein the non-motor symptom is at least one symptom selected from a group of symptoms consisting of cognitive disorder, attentional function disorder, cognitive memory disorder, working memory disorder, depressed mood, feeling of anxiety, feeling of urgency, apathy, psychological symptoms (hallucination, visual hallucination, delusion, and auditory hallucination), dopamine dysregulation syndrome, impulse control disorder, orthostatic hypotension, postural hypotension, urinary disorder, sexual dysfunction, digestive symptoms (drooling, swallowing, nausea, constipation), sleep disorder, pain and other paresthesia (olfactory abnormality and visual abnormality), weight change, fatigue, diaphoresis, and syncope.

### [Item 5]

The method according to item 4, wherein at least one symptom selected from the group of symptoms consisting of cognitive disorder, attentional function disorder, cognitive memory disorder, working memory disorder, depressed mood, feeling of anxiety, feeling of urgency, apathy, psychological symptoms (hallucination, visual hallucination, and auditory hallucination), dopamine dysregulation syndrome, impulse control disorder, orthostatic hypotension, postural hypotension, urinary disorder, sexual dysfunction, digestive symptoms (drooling, swallowing, nausea, constipation), sleep disorder, pain and other paresthesia (olfactory abnormality and visual abnormality), weight change, fatigue, diaphoresis, and syncope is attributable to Parkinson's disease.

### [Item 6]

The method according to any one of items 1 to 5 which is a method for treatment and/or prophylaxis of Parkinson's disease characterized by treating and/or preventing dyskinesia.

### [Item 7]

The method according to any one of items 1 to 6 is characterized by Parkinson's disease, according to Hoehn & Yahr severity level classification, being classified as one of level I, level II, level III, level IV, and level V.

### [Item 8]

The method according to any one of items 1 to 7 is characterized by irradiating the light of a specific wavelength constantly or at a specific blinking frequency to the subject.

### [Item 9]

The method according to any one of items 1 to 8 is characterized by the subject being administered or having administered at least one medical agent selected from a group consisting of levodopa, phenylalanine, tyrosine, bromocriptine mesylate, pergolide mesylate, cabergoline, talipexole hydrochloride, pramipexole hydrochloride, ropinirole hydrochloride, rotigotine, apomorphine hydrochloride hydrate, trihexyphenidyl hydrochloride, biperiden, selegiline, safinamide, rasagiline, entacapone, opicapone, droxidopa, istradefylline, quetiapine, zonisamide, and amantadine.

### [Item 10]

The method according to any one of items 1 to 9, wherein the light is violet light.

### [Item 11]

The method according to any one of items 1 to 10, wherein the specific wavelength includes a wavelength in a range of 360 nm to 400 nm.

### [Item 12]

The method according to any one of items 1 to 11, wherein the specific wavelength includes a wavelength of approximately 380 nm.

### [Item 13]

The method according to any one of items 1 to 12 is characterized by the light being one of light irradiated constantly and light blinking at a specific blinking frequency.

### [Item 14]

The method according to item 13, wherein the blinking frequency is in a range of 30 Hz to 70 Hz.

### [Item 15]

The method according to one of items 13 and 14, wherein the blinking frequency is in a range of 35 Hz to 60 Hz.

### [Item 16]

The method according to any one of items 13 to 15, wherein the blinking frequency is 40 Hz.

### [Item 17]

The method according to any one of items 1 to 16 is characterized by irradiating the light during daytime.

### [Item 18]

The method according to any one of items 1 to 17 is characterized by irradiating the light for 10 seconds or more, as a total time of light irradiation.

### [Item 19]

The method according to any one of items 1 to 18, wherein the light is irradiated such that irradiance of light incident on eyes is within a range of 0.5 µW/cm² to 1000 µW/cm².

### [Item 20]

The method according to any one of items 1 to 19 is characterized by irradiating the light for five minutes or more, as a total time of light irradiation.

### [Item 21]

The method according to any one of items 1 to 20, wherein the light irradiating apparatus is spectacles with a light source or a spectacle frame with a light source, a desktop light source, a mobile terminal mounted light source, a light source installed nearby or in front of face, a portable light source, an indoor lighting, and a desk lamp.

### [Item 22]

The method according to any one of items 1 to 21, wherein the subject is a human.

### [Item 23]

An apparatus for treatment and/or prophylaxis of Parkinson's disease by irradiating light of a specific wavelength constantly or at a specific blinking frequency to a subject is characterized by including a light source which irradiates light of a specific wavelength constantly or at a specific blinking frequency.

### [Item 24]

An apparatus according to item 23 is characterized by further including a controller which controls emission of light which induces specific brain waves same as, or substantially same as, or different from an irradiation state of light in the subject to which the light is irradiated.

### [Item 25]

The apparatus according to item 24, wherein the controller, by transceiving to and from an isolated controller such as a portable terminal, carries out control by changing irradiation conditions such as the blinking frequency of light of a specific wavelength, irradiance, irradiation time, irradiation-start time, and irradiation-end time.

### [Item 26]

The apparatus according to item 25 is characterized by further including a driving circuit which drives the light source.

### [Item 27]

The apparatus according to item 26,
wherein the driving circuit includes at least one processor and at least one memory for storing commands that are executable by the processor, and
the processor is communicably connected to the light source and the memory.

### [Item 28]

The apparatus according to any one of items 23 to 27, wherein the apparatus for treatment and/or prophylaxis of Parkinson's disease is characterized by treating and/or preventing motor symptoms and/or non-motor symptoms of Parkinson's disease.

### [Item 29]

The apparatus according to item 28, wherein the motor symptom is at least one symptom selected from a group of symptoms consisting of difficulty in speaking, ptyalism (excessive drooling), difficulty in chewing, difficulty in swallowing, difficulty in feeding action, difficulty in changing clothes, difficulty in maintaining personal hygiene, difficulty in writing, decrease in hobbies and entertainment, difficulty in turning over, tremor, difficulty in getting up from bed, chair, and wheelchair, difficulty in walking, loss of balance, freezing of gait (FOG), stiffness in walking, difficulty in speech, loss of facial expression, rigidity, finger tapping, impairment in hand movement, impairment in pronation and supination movement of hand, toe tapping, impairment in the agility of the lower extremities, impairment in postural stability, difficulty in maintaining posture, general spontaneity of movement (bradykinesia), hand posture tremor, hand movement tremor, resting tremor amplitude, resting tremor, and restless leg syndrome.

### [Item 30]

The apparatus according to one of items 28 and 29, wherein the non-motor symptom is at least one symptom selected from a group of symptoms consisting of cognitive disorder, attentional function disorder, cognitive memory disorder, working memory disorder, depressed mood, feeling of anxiety, feeling of urgency, apathy, psychological symptoms (hallucination, visual hallucination, delusion, and auditory hallucination), dopamine dysregulation syndrome, impulse control disorder, orthostatic hypotension, postural hypotension, urinary disorder, sexual dysfunction, digestive symptoms (drooling, swallowing, nausea, constipation), sleep disorder, pain and other paresthesia (olfactory abnormality and visual abnormality), weight change, fatigue, diaphoresis, and syncope.

### [Item 31]

The apparatus according to item 30, wherein at least one symptom selected from a group of symptoms consisting of cognitive disorder, attentional function disorder, cognitive memory disorder, working memory disorder, depressed mood, feeling of anxiety, apathy, psychological symptoms (hallucination, visual hallucination, delusion, and auditory hallucination), dopamine dysregulation syndrome, impulse control disorder, orthostatic hypotension, postural hypotension, urinary disorder, sexual dysfunction, digestive symptoms (drooling, swallowing, nausea, constipation), sleep disorder, pain and other paresthesia (olfactory abnormality and visual abnormality), weight change, fatigue, diaphoresis, and syncope is attributable to Parkinson's disease.

### [Item 32]

The apparatus according to any one of items 23 to 31, wherein the apparatus for treatment and/or prophylaxis of Parkinson's disease is characterized by treating and/or preventing dyskinesia.

### [Item 33]

The apparatus according to any one of items 23 to 32 is characterized by Parkinson's disease, according to Hoehn & Yahr severity level classification, being classified as one of level I, level II, level III, level IV, and level V.

### [Item 34]

The apparatus according to any one of items 23 to 33 is characterized by the subject being administered or having administered at least one medical agent selected from a group consisting of levodopa, phenylalanine, tyrosine, bromocriptine mesylate, pergolide mesylate, cabergoline, talipexole hydrochloride, pramipexole hydrochloride, ropinirole hydrochloride, rotigotine, apomorphine hydrochloride hydrate, trihexyphenidyl hydrochloride, biperiden, selegiline, safinamide, rasagiline, entacapone, opicapone, droxidopa, istradefylline, quetiapine, zonisamide, and amantadine.

### [Item 35]

The apparatus according to any one of items 23 to 34, wherein the light is violet light.

### [Item 36]

The apparatus according to any one of items 23 to 35, wherein the specific wavelength includes a wavelength in a range of 360 nm to 400 nm.

### [Item 37]

The apparatus according to any one of items 23 to 36, wherein the specific wavelength includes a wavelength of approximately 380 nm.

### [Item 38]

The apparatus according to any one of items 23 to 37, wherein the blinking frequency is in a range of 30 Hz to 70 Hz.

### [Item 39]

The apparatus according to any one of items 23 to 38, wherein the blinking frequency is in a range of 35 Hz to 60 Hz.

### [Item 40]

The apparatus according to any one of items 23 to 39, wherein the blinking frequency is 40 Hz.

### [Item 41]

The apparatus according to any one of items 23 to 40 is characterized by irradiating the light during daytime.

### [Item 42]

The apparatus according to any one of items 23 to 41 is characterized by irradiating the light for 10 seconds or more, as a total time of light irradiation.

### [Item 43]

The apparatus according to any one of items 23 to 42, wherein the light is irradiated such that irradiance of light incident on eyes is within a range of 0.5 µW/cm² to 1000 µW/cm².

### [Item 44]

The apparatus according to any one of items 23 to 43 is characterized by irradiating the light for five minutes or more, as a total time of light irradiation.

### [Item 45]

The apparatus according to any one of items 23 to 44, wherein the light irradiating apparatus is spectacles with a light source or a spectacle frame with a light source, a desktop light source, a mobile terminal mounted light source, a light source installed nearby or in front of face, and portable light source, an indoor lighting, and a desk lamp.

### [Item 46]

The apparatus according to any one of items 23 to 45, wherein light of different wavelength, sound, vibrations, magnetic field, and electric field are applied together with the irradiation of light of a specific wavelength.

### [Item 47]

A computer-readable medium which is a non-transitory computer-readable medium having commands stored therein, and which is capable of causing an apparatus according to any one of items 23 to 46 including a light source which irradiates light of a specific wavelength constantly or at a specific blinking frequency to a living body, and a controller which controls the blinking frequency of the light source, to execute the following steps when a command is executed by a processor:
a step of operating the apparatus such that the controller controls the blinking frequency of the light source to 0 Hz or to a frequency in a range of 30 Hz to 75 Hz, and
a step of operating the apparatus such that the light source irradiates light of a wavelength in a range of 360 nm to 400 nm to a living body.

### [Item 48]

An instrument for treatment and/or prophylaxis of Parkinson's disease by light stimulation includes a glass, spectacle lens, or a contact lens which allows violet light to pass through.

Specific examples of the motor symptoms of Parkinson's disease treated and/or prevented by the present invention include difficulty in speaking, ptyalism (excessive drooling), difficulty in chewing, difficulty in swallowing, difficulty in feeding action, difficulty in changing clothes, difficulty in maintaining personal hygiene, difficulty in writing, decrease in hobbies and entertainment, difficulty in turning over, tremor, difficulty in getting up from bed, chair, and wheelchair, difficulty in walking, loss of balance, freezing of gait (FOG), stiffness in walking, difficulty in speech, loss of facial expression, rigidity, finger tapping, impairment in hand movement, impairment in pronation and supination movement of the hand, toe tapping, impairment in agility of the lower extremities, impairment in postural stability, difficulty in maintaining posture, general spontaneity of movement (bradykinesia), hand posture tremor, hand movement tremor, resting tremor amplitude, resting tremor, and restless leg syndrome.

Specific examples of the non-motor symptoms of Parkinson's disease treated and/or prevented by the present invention include cognitive disorder, attentional function disorder, cognitive memory disorder, working memory disorder, depressed mood, feeling of anxiety, feeling of urgency, apathy, psychological symptoms (hallucination, visual hallucination, delusion, and auditory hallucination), dopamine dysregulation syndrome, impulse control disorder, orthostatic hypotension, postural hypotension, urinary disorder, sexual dysfunction, digestive symptoms (drooling, swallowing, nausea, constipation), sleep disorder, pain and other paresthesia (olfactory abnormality and visual abnormality), weight change, fatigue, diaphoresis, and syncope.

More preferable examples of the non-motor symptoms of Parkinson's disease treated and/or prevented by the present invention include cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis.

Even more preferable example of the non-motor symptoms of Parkinson's disease treated and/or prevented by the present invention include visual hallucination, auditory hallucination, sleep disorder, and cognitive memory disorder.

A specific example of a symptom of Parkinson's disease treated and/or prevented by the present invention includes dyskinesia of a patient suffering from Parkinson's disease, or in other words, a time of occurrence of dyskinesia, an effect of dyskinesia on functions, a time spent during off-state, an effect of symptom fluctuation on functions, a complexity of motor symptom fluctuation, and dystonia in a painful off-state.

In the context of the present disclosure, the patients suffering from Parkinson's disease may include any one of patients classified as level I, level II, level III, level IV, and level V according to Hoehn & Yahr severity level classification.

When applying the method according to the present disclosure, the subject may be a patient who is being administered or was administered at least one antiparkinsonian drug.

Examples of antiparkinsonian drugs includes at least one or more medical agent selected from a group consisting of dopaminergic precursors, dopamine agonists, anticholinergic agents, monoamine oxidase B inhibitors (MAO-B inhibitors), COMT inhibitors (catechol-O-methyltransferase inhibitors), noradrenaline precursors, adenosine A2A antagonists, antipsychotic agents, zonisamide, and/or amantadine.

Specific examples of dopaminergic precursor include phenylalanine, tyrosine, and levodopa (L-dopa).

Specific examples of dopamine agnostic include bromocriptine mesylate, pergolide mesylate, cabergoline, talipexole hydrochloride, pramipexole hydrochloride, ropinirole hydrochloride, rotigotine, and apomorphine hydrochloride hydrate.

Specific examples of anticholinergic agent include trihexyphenidyl hydrochloride and biperiden.

Specific examples of monoamine oxidase B inhibitor (MAOB inhibitor) include selegiline, safinamide, and rasagiline.

Specific examples of COMT inhibitor include entacapone and opicapone.

Specific examples of noradrenaline precursor include droxidopa.

Specific examples of adenosine A2A antagonist include istradefylline.

Specific examples of antipsychotic agent include quetiapine.

Moreover, according one aspect, the present disclosure is related to a method for treatment and/or prophylaxis of Parkinsons disease in a subject which requires the treatment and/or prophylaxis, which includes suppressing or improving deposition of α-synuclein and/or p62 protein in the brain by irradiating light of a specific wavelength to the subject. In some embodiments, the present disclosure is related to a method which includes irradiating light of a specific wavelength to a subject and suppressing or improving deposition of α-synuclein and/or p62 protein in the brain of a patient suffering from Parkinson's disease.

Moreover, according to one aspect, the present disclosure is related to a method for accelerating proliferation and/or differentiation of oligodendrocytes in a patient suffering from Parkinson's disease requiring treatment and/or prophylaxis, which includes irradiating light of a specific wavelength irradiating light of a specific wavelength to the patient. Moreover, in some embodiment, the present disclosure is related to a method for treatment and/or prophylaxis of Parkinsons disease in a subject which requires the treatment and/or prophylaxis, which includes irradiating light of a specific wavelength to the subject, and accordingly, accelerating proliferation and/or differentiation of oligodendrocytes in the subject, thereby treating and/or preventing Parkinson's disease in the subject. It has been known that in Parkinson's disease, oligodendrocytes which are glial cells and an amount of differentiated myelin decrease, and accelerating the proliferation and differentiation of oligodendrocytes is considered to be significant for treatment and/or prophylaxis of Parkinson's disease.

In some embodiments, the light used is violet light.

In some embodiments, the specific wavelength used includes a wavelength in a range of 360 nm to 400 nm, and particularly includes a wavelength of approximately 380 nm.

In some embodiments, the blinking frequency used is 0 Hz or a frequency in range of 30 Hz to 70 Hz, and is particularly 0 Hz or a frequency in a range of 35 Hz to 60 Hz, and more specifically is 0 Hz or approximately 40 Hz for example.

In some embodiments, irradiation conditions further include an irradiation time of the light source.

In some embodiments, the light source may be spectacles with a light source or a spectacle frame with a light source, a desktop light source, a mobile terminal mounted light source, a light source installed nearby or in front of face, a portable light source, an indoor lighting, and a desk lamp.

Preferable examples of the light source include spectacles with a light source or a spectacle frame with a light source, a desktop light source, a mobile terminal mounted light source, a light source installed nearby or in front of face, a portable light source, and a desk lamp.

More preferable examples of the light source include spectacles with a light source or a spectacle frame with a light source, a desktop light source, a mobile terminal mounted light source, or a light source installed nearby or in front of face.

Even more preferable examples of the light source include spectacles with a light source or a spectacle frame with a light source or a desktop light source.

The most preferable examples of the light source include spectacles with a light source or a spectacle frame with a light source.

In some embodiments, the subject to which the present disclosure is applied is a human.

According to one aspect, the present disclosure is related to an apparatus for treatment and/or prophylaxis of Parkinson's disease by light stimulation which includes at least one light source which emits light and a driving circuit which drives the light source, and the light emitted by the light source is light of a specific wavelength which produces an effect of treatment and/or prophylaxis of Parkinson's disease by being irradiated to a living body.

Moreover, according to one aspect, the present disclosure is related to an apparatus for treatment and/or prophylaxis of the motor symptoms, the non-motor symptoms and/or other symptoms by irradiating light to a living body, which includes at least one light source which emits light and a driving circuit which drives the light source.

In some embodiments, the driving circuit in the apparatus according to the present disclosure includes at least one processor which is communicably connected to the light source and at least one memory, and at least one memory for storing commands executable by the processor.

According to one aspect, the present disclosure is related to a method of operating an apparatus according to the present disclosure which includes a light source which irradiates light of a specific wavelength constantly or at a specific blinking frequency to a living body and a controller which controls the blinking frequency of the light source, which includes a step of controlling the blinking frequency of the light source to 0 Hz or to a frequency in a range of 30 Hz to 75 Hz by the controller and a step of irradiating light of a wavelength in a range of 360 nm to 400 nm by the light source.

According to one aspect, the present disclosure is related to a computer program which causes an apparatus including a light source which irradiates light of a specific wavelength constantly or at a specific blinking frequency to a living body and a controller which controls the blinking frequency of the light source, to execute the method of operating according to the present disclosure.

In the method and apparatus according to the present disclosure, the light is violet light for example. According to the present invention, since it is possible to irradiate violet light to a living body, it is possible to have an effect on a living body without feeling flickering or dazzling as with white light. Violet light is light having a wavelength in a range of 360 nm to 400 nm, and light with this wavelength has a relative visibility lower as compared to that of white light, and is a wavelength region which imparts no uncomfortable feeling or hardly imparts any uncomfortable feeling to a living body (a human in particular).

In some embodiments of the present disclosure, light of a wavelength in a range of 350 nm to 400 nm, for example, light of any one of the wavelengths of 350 nm, 360 nm, 370 nm, 380 nm, 390 nm or 400 nm, or light of an arbitrary wavelength included in a range stipulated by the abovementioned arbitrary wavelength (for example, a range of 370 nm to 390 nm) may be used. In some embodiments of the present disclosure, the wavelength includes a wavelength of approximately 380 nm. The term 'approximately' used in the preset specification refers to any value that is within a variation of up to ±5% of the value modified by the term.

In the method and apparatus according to the present disclosure, the irradiation condition of light is either irradiated constantly (in other words, 0 Hz) or the blinking frequency in a range of more than 0 Hz up to 150 Hz.

In some embodiments of the present disclosure, the constantly irradiated light (0 Hz) or light of a blinking frequency in a range of 30 Hz to 75 Hz, for example, light of any one of the blinking frequencies 30 Hz, 35 Hz, 40 Hz, 45 Hz, 50 Hz, 55 Hz, 60 Hz, 65 Hz, 70 Hz or 75 Hz, or light of an arbitrary blinking frequency included in a range stipulated by the abovementioned arbitrary blinking frequency (for example, a range of 35 Hz to 45 Hz) may be used. In some embodiments of the present disclosure, the blinking frequency is approximately 40 Hz.

In the method and apparatus according to the present disclosure, it is possible to irradiate light such that irradiance of light incident on eyes is, for example, in a range of 0.5 µW/cm² to 1000 µW/cm², or in a range of 0.1 µW/cm² (0.001 W/m²) to 5000 µW/cm² (50W/m²). According to some embodiments, it is believed that by irradiating light such as violet light in the abovementioned range of irradiance, it is possible to have an effect on the central nervous system. Violet light, in general, even being a weak light (low intensity light) of small amount (light having a weak optical sensitivity), has been confirmed to be capable of causing a characteristic phenomenon.

In the method and apparatus according to the present disclosure, the controller of the apparatus is capable of carrying out the control by changing irradiation conditions such as irradiation state of the light (including irradiating constantly or irradiating at a blinking frequency), irradiance, irradiation time, irradiation-start time, irradiation-end time, constant irradiation, or blinking frequency by transceiving to and from an isolated controller such as a portable terminal. According to some embodiments, since an isolated control of the abovementioned various irradiation conditions is carried out, it is possible to achieve a desired effect by setting arbitrarily appropriate irradiation conditions for producing an effect of treating and/or preventing at least one symptom selected from a group of motor symptoms and non-motor symptoms, or in other words, cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis.

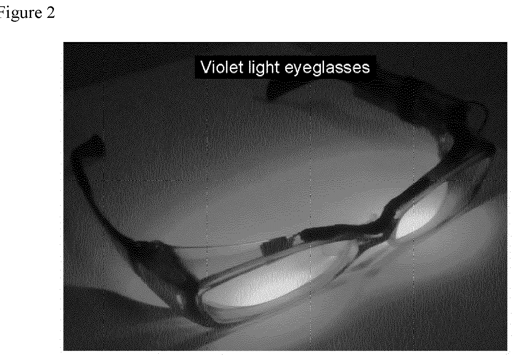

In the method and apparatus according to the present disclosure, the light source may be spectacles with a light source (refer to Fig. 2 for example) or a spectacle frame with a light source, a desktop light source, a mobile terminal mounted light source, or a light source installed nearby or in front of face. According to some embodiments, since it is possible to irradiate specific light from a light source installed nearby or in front of face such as spectacles with a light source or a spectacle frame with a light source that can be worn easily and have no uncomfortable feeling in daily use, they are highly practical, and are capable of irradiating light any time even in various situations and varied environments.

In the method and apparatus according to the present disclosure, the light source may be a non-mounting type light source such as a portable light source, or may be a mounting type light source such as an indoor lighting, a table stand, and a dedicated device. According to some embodiments, it is possible to make it an apparatus in various forms of light source appropriate for an environment of usage. For instance, the light source may be used in combination with a glass, a spectacle lens, or a contact lens that allows violet light to pass through. Moreover, sunlight that has passed through a glass, a spectacle lens, or a contact lens that allows violet light to pass through may be used as the light source.

The method by light stimulation according to the present disclosure is a method for treatment and/or prophylaxis of Parkinson's disease by irradiating light of a specific wavelength constantly or at a specific blinking frequency to a living body (for example, mammals including a human), and is characterized by controlling emission of light which improves or suppresses at least one symptom selected from a group of motor symptoms and non-motor symptoms in a living body having received the light, or in other words, cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis.

The apparatus according to the present disclosure is an apparatus for treatment and/or prophylaxis of Parkinson's disease by irradiating violet light constantly to a living body, and is characterized by including a light source which emits violet light and an emission time controller which irradiates the violet light for a specific time or a specific period of time.

Furthermore, as it is described below while referring to the accompanying diagrams, in some embodiments of the present disclosure, a method for treatment and/or prophylaxis of Parkinson's disease in the subject which requires treatment and/or prophylaxis, a method for treatment and/or prophylaxis of at least one symptom selected from a group of symptoms consisting of motor symptoms and non-motor symptoms, or in other words, cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis in the subject which requires treatment and/or prophylaxis, an apparatus for treatment and/or prophylaxis of Parkinson's disease by light stimulation, an apparatus for treatment and/or prophylaxis by light stimulation of at least one symptom selected from a group of symptoms consisting of motor symptoms and non-motor symptoms, or in other words, cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis, a method of operating the apparatus, and a computer program which executes the method of operating are provided.

### Brief Description of the Drawings

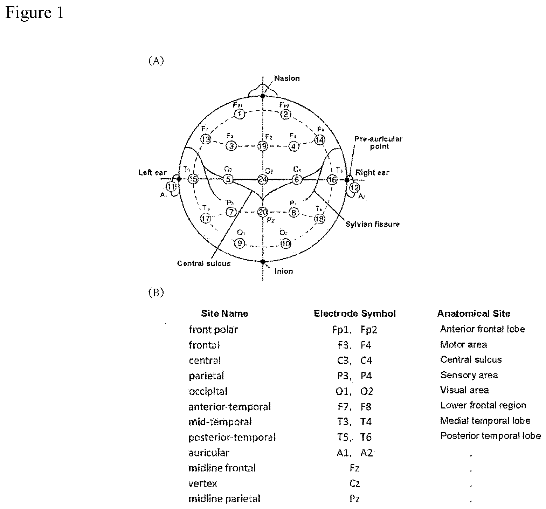

Fig. 1 is a schematic diagram of various parts of brain:
Fig. 2 is an example of violet light spectacles which irradiate violet light;
Fig. 3 is a graph showing a relationship of wavelength and spectral irradiance of violet fluorescent light;
Fig. 4 is a light spectrum of an LED with a peak wavelength of 375 nm;
Fig. 5 is a block diagram of an embodiment of an apparatus for controlling biological function according to the present invention;
Fig. 6 is a micrograph showing that a formation of aggregate including α-synuclein is suppressed by VL; and
Fig. 7 is a micrograph showing that a formation of aggregate including p62 is suppressed by VL.

### Mode for Carrying Out the Invention

A method for treatment and/or prophylaxis of Parkinson's disease in a subject which requires treatment and/or prophylaxis, a method for treatment and/or prophylaxis of at least one symptom selected from a group of symptoms consisting of motor symptoms and non-motor symptoms, or in other words, cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis in the subject which requires treatment and/or prophylaxis, an apparatus for treatment and/or prophylaxis of Parkinson's disease by light stimulation, an apparatus for treatment and/or prophylaxis of at least one symptom selected from a group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis, a method of operating the apparatus, and a computer program which executes the method of operating will be described while referring to the accompanying diagrams. The present invention is not restricted to following embodiments and examples, and includes various modified examples and application examples within the scope of the present invention.

### [Method for treatment and/or prophylaxis of Parkinson's disease]

A method for treatment and/or prophylaxis of Parkinson's disease according to the present disclosure includes irradiating light of a specific wavelength to a subject which requires the treatment and/or prophylaxis.

The inventors of the present invention discovered that by irradiating light of a specific wavelength to a subject, it is possible to improve symptoms such as visual hallucination of Parkinson's disease. Therefore, one aspect of the present disclosure is related to a method for treatment and/or prophylaxis of Parkinson's disease in a subject which requires the treatment and/or prophylaxis, and this method includes improving by irradiating light of a specific wavelength to the subject, at least one symptom selected from a group of symptoms consisting of motor symptoms and non-motor symptoms, or in other words, cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis. Here, the subject may be a human suffering from Parkinson's disease or a human at risk of developing Parkinson's disease.

As symptoms of Parkinson's disease, examples of motor symptoms include resting tremor, akinesia, muscle stiffness, and impairment of postural reflex, and examples of non-motor symptoms include autonomic nervous symptoms, cognitive disorder, olfactory disturbance, sleep disorder, psychological symptoms (symptoms such as depression and anxiety, visual hallucination or illusion, delusion), fatigue, pain, and weight loss.

In diagnosis of Parkinson's disease, firstly, a check of whether or not there is any motor symptom is made, and then symptoms supportive of Parkinson's disease and symptoms supportive of a disease other than Parkinson's disease are checked and a judgment of whether it is Parkinson's disease or a disease other than Parkinson's disease is made.

The patient suffering from Parkinson's disease, according to Hoehn & Yahr severity level classification, may be classified as level I, level II, level III, level IV, and level V (level I: tremor of hand and leg and stiffening of muscles is observed only in one side of body. There is no body impairment or a minor impairment if at all any; level II: tremor of both hands and legs and stiffening of muscles in both sides is observed; level III: Taking short steps while walking, freezing of gait (FOG) is observed; level IV: Getting up, walking becomes difficult; level V: wheelchair becomes necessary). Moreover, for evaluation of Parkinson's disease, UPDRS (Unified Parkinson's Disease Rating Scale) may be used. UPDRS is a unified evaluation criteria for Parkinson's disease (PD) announced in 1987, and constitutes a total of 42 evaluation items in four parts (part I: mentation, behavior, and mood; part II: activities of daily life ADL; part III: motor sections; part IV: complications of therapy). In the context of the present disclosure, an effectiveness of the treatment of Parkinson's disease can be based on these criteria. The effectiveness of the treatment of Parkinson's disease may be evaluated based on PDQ-39 which is a disease-specific QoL (Quality of Life) criteria. PDQ-39 is composed of 39 questions in 8 domains, and the higher the value, the lower is the QoL.

As mentioned above, one aspect of the present disclosure is related to a method for treatment and/or prophylaxis of Parkinson's disease in a subject which requires the treatment and/or prophylaxis, and the method includes irradiating light of a specific wavelength to the subject. Moreover, the method may include treatment and/or prophylaxis of Parkinson's disease in the subject by improving or preventing at least one symptom selected from motor symptoms and non-motor symptoms in the subject, or in other words, cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis by irradiating light of a specific wavelength to the subject.

At the present stage, supportive measures which alleviate the symptoms is the main focus in the treatment of Parkinson's disease. The basis of the treatment is drug therapy, and as a medication, medical agents such as a dopamine precursor (levodopa; L-dopa) and a dopamine agonist (such as bromocriptine mesylate, pergolide mesylate, cabergoline, talipexole hydrochloride, pramipexole hydrochloride, ropinirole hydrochloride, rotigotine, and apomorphine hydrochloride hydrate), an anticholinergic agent (such as trihexyphenidyl hydrochloride and biperiden), a monoamine oxidase B inhibitor (such as selegiline, safinamide, and rasagiline), a COMT inhibitor (such as entacapone and opicapone), a noradrenaline precursor (such as droxidopa), an adenosine A2A antagonist (such as istradefylline), an antipsychotic agent (such as quetiapine and amantadine), zonisamide, and amantadine are used. Therefore, the method for treatment and/or prophylaxis according to the present disclosure may be applied to a patient who is being administered or was administered these medical agents. The method for treatment and/or prophylaxis according to the present disclosure may be applied in combination with administering of these medical agents.

MMSE (Mini-Mental State Examination) for example, may be used for evaluation of cognitive memory disorder and working memory disorder. MMSE is composed of 11 questions, and the subject answers each of these questions one by one posed by the questioner. Time limit for each question is 10 seconds, and when the time limit is over, the next question is posed. The examination has a maximum score of 30 points, and score of 23 points or less may be judged to be 'suspected dementia' and a score in a range of 24 to 27 points may be judged to be 'suspected mild cognitive impairment (MCI)'. Hasegawa Dementia Scale - Revised (HDS-R) may be used instead of MMSE or in addition to MMSE. HDS-R is composed of nine questions, and as compared to MMSE, more emphasis is placed on memory.

From the research of a molecular level, it has become evident that deposition of α-synuclein and p62 protein is observed in the brain of patients suffering from Parkinson's disease. The inventors of the present invention discovered that it is possible to suppress the deposition of α-synuclein and p62 protein in the brain by irradiating light of a specific wavelength to the subject. Therefore, one aspect of the present disclosure is related to a method for suppressing or improving the deposition of α-synuclein and p62 protein in the brain of a patient suffering from Parkinson's disease, which includes irradiating light of a specific wavelength to the subject.

From the research of cellular level, it has become evident that oligodendrocytes which are glial cells and an amount of differentiated myelin decrease in Parkinson's disease. The inventors of the present invention discovered that it is possible to accelerate the proliferation and/or differentiation of oligodendrocytes by irradiating light of a specific wavelength to the subject. Therefore, one aspect of the present disclosure is related to a method for accelerating proliferation and/or differentiation of oligodendrocytes in a patient suffering from Parkinson's disease requiring the treatment and/or prophylaxis, which includes irradiating light of a specific wavelength to the patient.

In some embodiments of the method according to the present disclosure, the light used may be violet light. Moreover, in some embodiments of the present disclosure, the specific wavelength used includes a wavelength in a range of 360 nm to 400 nm, and can particularly include a wavelength of approximately 380 nm. The blinking frequency, for instance, may be 0 Hz or a frequency in a range of 30 Hz to 70 Hz, and particularly 0 Hz or a frequency in a range of 35 Hz to 60 Hz. The blinking frequency in particular, may be 0 Hz or approximately 40 Hz. The irradiation conditions may further include an irradiation time of the light source. The light source may be spectacles with a light source or a spectacle frame with a light source, a desktop light source, for example, a desktop light placed at a bed side, a desk lighting, a mobile terminal mounted light source, a light source installed nearby or in front of face, a portable light source, an indoor lighting, a wall-mounted light, a ceiling light, and a desk lamp, but it not restricted to these light sources.

In some embodiments of the present disclosure, the specific time of irradiating light is, for example, 10 seconds or more, 20 seconds or more, 30 seconds or more, 40 seconds or more, 50 seconds or more, one minute or more, three minutes or more, five minutes or more, 10 minutes or more, 15 minutes or more, 30 minutes or more, one hour or more, two hours or more, three hours or more, four hours or more, five hours or more, 10 hours or more, 20 hours or more, 30 hours or more, 40 hours or more, 50 hours or more, 100 hours or more 150 hours or more, 200 hours or more, 250 hours or more, or 300 hours or more, as a total time of irradiating light. Moreover, the specific time of irradiating light is, for example, 100000 hours or less, 10000 hours or less, 1000 hours or less, 500 hours or less, 300 hours or less, or 100 hours or less. The specific time of irradiating light, for example, the time of irradiating continuously, is 10 seconds or more, 20 seconds or more, 30 seconds or more, 40 seconds or more, 50 seconds or more, one minute or more, three minutes or more, five minutes or more, 10 minutes or more, 15 minutes or more, 30 minutes or more, one hour or more, two hours or more, three hours or more, four hours or more, five hours or more, 10 hours or more, or 20 hours or more. Moreover, the specific time of irradiating light, for example, the time of irradiating continuously, is 20 hours or less, 10 hours or less, five hours or less, four hours or less, three hours or less, two hours or less, one hour or less, 30 minutes or less, 15 minutes or less, 10 minutes or less, five minutes or less, three minutes or less, one minute or less, 50 seconds or less, 40 seconds or less, 30 seconds or less, or 20 seconds or less. Moreover, the specific time of irradiating light may be any arbitrary time in a range 10 seconds to 24 hours per day such as, 10 seconds, 30 seconds, 45 seconds, one minute, three minutes, five minutes, 10 minutes, 15 minutes, 30 minutes, 45 minutes, one hour, two hours, three hours, four hours, five hours, six hours, seven hours, eight hours, nine hours, 10 hours, 11 hours, 12 hours, 18 hours, and 24 hours, or may be an arbitrary time included in a range stipulated by the abovementioned arbitrary time (for example, a range of 1 hour to 12 hours). The specific period of irradiating the light continuously may be a period of one day or more, two days or more, three days or more, four days or more, five days or more, six days or more, one week or more, two weeks or more, three weeks or more, one month or more, two months or more, three months or more, six months or more, one year or more, two years or more, three years or more, or a period longer than these periods. The specific period of irradiating the light continuously may be for example, 10 years or less, five years or less, one year or less, six months or less, three months or less, two months or less, one month or less, three weeks or less, two weeks or less, one week or less, six days or less, five days or less, four days or less, three days or less, or two days or less.

One aspect of the present disclosure is related to a method for treatment and/or prophylaxis of Parkinson's disease in a subject requiring the treatment and/or prophylaxis by improving or suppressing at least one symptom selected from a group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis by irradiating light of a specific wavelength to the subject by controlling the light irradiating apparatus. In some embodiments, the light irradiating apparatus is capable of irradiating violet light. Moreover, in some embodiments, the light irradiating apparatus is capable of irradiating light of a wavelength in a range of 360 nm to 400 nm, and particularly, light of a wavelength of approximately 380 nm. The light irradiating apparatus may be capable of controlling the blinking frequency to 0 Hz or to a frequency in a range of 30 Hz to 70 Hz, and particularly to 0 Hz or to a frequency in a range of 35 Hz to 60 Hz, and the blinking frequency may be controlled in particular to 0 Hz or approximately 40 Hz. The light irradiating apparatus may be an apparatus which is capable of controlling the irradiation time. The light source may be in the form of spectacles with a light source or a spectacle frame with a light source, a desktop light source, a mobile terminal mounted light source, a light source installed nearby or in front of face, a portable light source, an indoor lighting, and a desk lamp, but is not restricted to thereto.

### [Apparatus]

One aspect of the present disclosure is related to an apparatus for treatment and/or prophylaxis of Parkinson's disease by light stimulation. In some embodiments, the apparatus for treatment and/or prophylaxis of Parkinson's disease according to the present disclosure may include at least one light source which emits light and a driving circuit which drives the light source. Here, the light emitted by the light source is light of a specific wavelength which produces an effect of treatment and/or prophylaxis of Parkinson's disease by being irradiated to a living body.

Moreover, one aspect of the present disclosure is related to an apparatus for treatment and/or prophylaxis of a disorder which treats and/or prevents by a dopamine precursor (levodopa; L-dopa) and a dopamine agonist (such as bromocriptine mesylate, pergolide mesylate, cabergoline, talipexole hydrochloride, pramipexole hydrochloride, ropinirole hydrochloride, rotigotine, and apomorphine hydrochloride hydrate), an anticholinergic agent (such as trihexyphenidyl hydrochloride and biperiden), a monoamine oxidase B inhibitor (MAOB inhibitor) (selegiline, safinamide, and rasagiline), a COMT inhibitor (such as entacapone and opicapone), a noradrenaline precursor (droxidopa), an adenosine A2A antagonist (such as istradefylline), zonisamide, amantadine, and an antipsychotic agent (such as quetiapine), by light stimulation. In some embodiments, the apparatus for treatment and/or prophylaxis of at least one symptom selected from a group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis according to the present disclosure may include at least one light source which emits light and a driving circuit which drives the light source. Here, the light emitted by the light source is light which, by being irradiated to a living body, produces an effect of accelerating improvement or suppression of at least one symptom selected from the group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis.

In the present specification, the apparatus for treatment and/or prophylaxis of Parkinsons disease by light stimulation which is an apparatus for treatment and/or prophylaxis of at least one symptom selected from a group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis by light stimulation is sometimes collectively referred to as 'apparatus for controlling biological function' or simply 'apparatus'.

These apparatuses are considered to be capable of applying stimulus to central nerve of a living body by controlling a radiation state of light. As described above, improvement or suppression of at least one symptom selected from a group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis in a patient subjected to light stimulation, thereby leading to treatment and/or prophylaxis of Parkinson's disease.

In some embodiments, the apparatus according to the present disclosure, as mentioned above, is an apparatus for treatment and/prophylaxis of Parkinson's disease by accelerating improvement or suppression of at least one symptom from a group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis by irradiating violet light constantly or at a specific blinking frequency to a living body, and is characterized by including a light source which emits the violet light, a light-emission cycle controller which makes irradiate the violet light constantly or at a specific blinking frequency, and a light-emission time controller which makes irradiate the violet light for a specific time or a specific period of time, and being an apparatus used for treatment and/or prophylaxis of the disorder.

Moreover, the apparatus according to the present disclosure, in some embodiments, is an apparatus which controls biological function related to Parkinson's disease by irradiating violet light constantly to a living body, and is characterized by including a light source which emits the violet light and a light-emission time controller which makes irradiate the violet light for a specific time or a specific period of time.

Moreover, the apparatus according to the present disclosure, in some embodiments, is related to an apparatus used for treatment and/or prophylaxis of the abovementioned disorder by light stimulation which includes at least one light source which emits light, and a driving circuit which drives the light source, and the light emitted by the light source is light of a specific wavelength which produces the effect by being irradiated to a living body. Therefore, one aspect of the present disclosure is related to an apparatus for treatment and/or prophylaxis of Parkinson's disease by light stimulation which is configured to include at least one light source which emits light and a driving circuit which drives the light source, and the driving circuit includes at least one processor which is communicably connected to the light source and at least one memory, and at least one memory for storing commands that are executable by the processor, and the light source emits light of a specific wavelength which produces an effect of treating and/or preventing Parkinson's disease by being irradiated to a living body. Moreover, one aspect of the present disclosure is related to an apparatus for treatment and/or prophylaxis of at least one symptom selected from a group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis by light stimulation, which is configured to include at least one light source which emits light and a driving circuit which drives the light source, and the driving circuit includes at least one processor which is communicably connected to the light source and at least one memory, and at least one memory which is for storing commands that are executable by the processor, and the light source emits light of a specific wavelength which produces an effect of treating and/or prohibiting at least one symptom selected from the group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis by being irradiated to a living body.

### (Light source)

The wavelength of light emitted by the light source is not restricted in particular, and in some embodiments, violet light defined by a wavelength range of 360 nm to 400 nm is used.

A light source with a possible oscillating frequency in a range of 0 Hz (constantly irradiated light, direct light) to 150 Hz can be used preferably. The frequency can be adjusted in units of 0.5 Hz and 1 Hz by setting of the controller, and light of an arbitrary blinking frequency can be produced. As the blinking frequency is increased, there is an advantage that the blinking is not sensed any more, although this perception varies from person to person. The blinking frequency is not restricted to 40 Hz that was used in experimental examples.

The irradiance from the light source may be variable or may be a constant value. Although irradiance with maximum output of 310 µW/cm² is used in some embodiments, it is not restricted to this value. The light source can be configured arbitrarily for irradiance in a range of 0.1 µW/cm² (0.001 W/m²) to 5000 µW/cm² (50 W/m²) for example, or irradiance in a range of 1 µW/cm² (0.01 W/m²) to 1000 µW/cm² (10 W/m²) for example, or irradiance in a range of 0.5 µW/cm² (0.005 W/m²) to 500 µW/cm² (5 W/m²) for example, or irradiance in a range of 0.5 µW/cm² to 1000 µW/cm², or irradiance in a range of 5 µW/cm² to 1000 µW/cm² for example, or irradiance in a range of 10 µW/cm² to 1000 µW/cm² for example, or irradiance in a range of 100 µW/cm² to 500 µW/cm² for example, or irradiance in a range of 200 µW/cm² to 400 µW/cm². Moreover, example of irradiance from the light source include, irradiation intensity of light incident on eyes in a range of 0.1 µW/cm² (0.001 W/m²) to 5000 µW/cm² (50 W/m²) for example, or in a range of 1 µW/cm² (0.01 W/m²) to 1000 µW/cm² (10 W/m²) for example, or in a range of 0.5 µW/cm² (0.005 W/m²) to 500 µW/cm² (5 W/m²) for example, or in a range of 0.5 µW/cm² to 1000 µW/cm², or in a range of 5 µW/cm² to 1000 µW/cm² for example, or in a range of 10 µW/cm² to 1000 µW/cm² for example, or in a range of 100 µW/cm² to 500 µW/cm² for example, or in a range of 200 µW/cm² to 400 µW/cm². Furthermore, since a light source with such irradiance can be used easily for spectacles or a spectacle frame, and other portable irradiation equipment, it can be worn even in day-to-day life. Even with weak light of extremely small amount (light with weak optical intensity) in particular, an occurrence of characteristic phenomenon has been verified, and an effect on various parts of a living body including brain, and an application in cell activity (used with a significance including gene expression control) can be anticipated.

Light may be specified in terms of relative luminosity. Since characteristics of the present invention can be realized even with a low relative luminosity, the violet light which generates stimulation in a living body can be irradiated while blinking, at a low relative luminosity, and a desired site can be stimulated without strain on a living body.

It is preferable to set the irradiation time of light arbitrarily according to the purpose thereof, and it may be a short time or a long time. The light can be irradiated intermittently (at regular interval or irregular interval) or continuously. The time of irradiation of light can be set to be a period of time between 8 am to 1 pm, between 9 am to 12 pm, between 10 am to 11 am, and the period can be set to be at least 10 minutes, at least 15 minutes, at least 20 minutes, at least 30 minutes, at least one hour, at least two hours, at least three hours, at least four hours, or at least five hours. In some embodiments, irradiation for a period of three hours from 9 am to 12 pm, or for a period of two hours from 9 am to 11 am is used. In a case of irradiation in which the time has been set in this manner, a timer function can be used.

The light source may be spectacles or a spectacle frame with a light source. Such spectacles or spectacle frame being easy to wear, and having a light source which emits light at a blinking frequency fitted to spectacles or spectacle frame with no uncomfortable feeling on a daily basis, they are highly practical, and can be worn any time even in various situations and varied environments. Moreover, the light source may be a desktop light source, or a light source installed nearby or in front of face such as a mobile terminal mounted light source, or a non-mounting type light source such as a portable light source, or may be a mounting type light source such as an indoor lighting, a table stand, and a dedicated device, and can be an apparatus in various forms of light source appropriate for an environment of usage.

### (Controller)

The controller is a section which controls irradiation conditions (irradiate constantly or at a blinking frequency) of light from the light source. The controller may be provided with an electric power source for supplying electric power to the light source, and such electric power source may be a battery, or may have a wire drawn to a battery installed at a different location. **In** a case of being immovable at one location, it may be connected to a household electric power supply.

It is preferable that the controller, by tranceiving to and from an isolated controller such as a portable terminal, changes irradiation conditions of light such as blinking frequency, irradiance, irradiation time, irradiation-start time, and irradiation-end time. Since such controller carries out an isolated control of various irradiation conditions mentioned above, it is possible to have the desired effect by setting arbitrarily the irradiation conditions appropriate for controlling the desired biological function.

Furthermore, the controller may have controller functions and timer functions of the light source. Examples of the controller functions include functions such as changing the frequency and irradiance, and setting the irradiation time. Moreover, examples of the timer functions include an ability to set the irradiance time. Such controller functions and timer functions may have been provided integrally with the instrument, or may be provided as separate members.

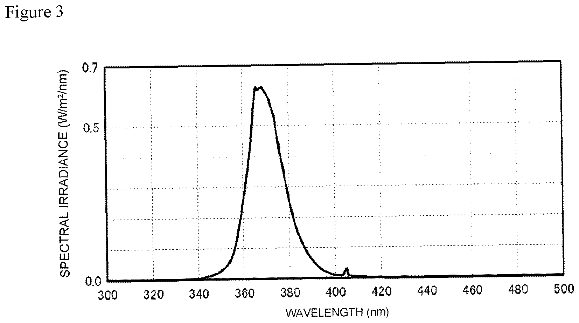

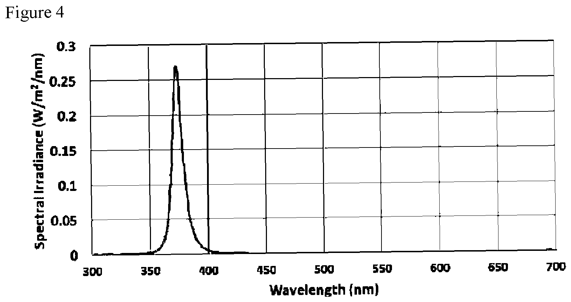

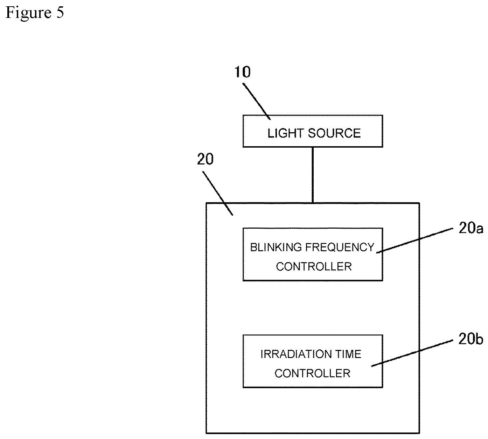

A block diagram of a simplified example of an apparatus which can be used for the abovementioned control of biological functions as an embodiment of the apparatus according to the present disclosure is shown in Fig. 5. The apparatus shown in Fig. 5 may include various functions of the apparatus for treatment and/or prophylaxis of Parkinson's disease by light stimulation and the apparatus for treatment and/or prophylaxis of at least one symptom selected from a group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis by light stimulation (these apparatuses can be collectively referred to as 'apparatus for controlling biological function') described in the present specification, and therefore, all the abovementioned apparatuses in the present specification can be represented by the block diagram in Fig. 5. The apparatus for controlling biological function can have a light source 10 and a controller 20. The light source 10 irradiates light of a specific wavelength. It is preferable that a wavelength of light irradiated by the light source 10 includes VL (violet light) or a wavelength range of 360 nm to 400 nm described heretofore, and more preferably, includes a wavelength of 380 nm. The light source 10 may be an arbitrary light source, and a light emitting diode (LED) can be used preferably from viewpoints such as small size, long life, and ease of blinking control (refer to Fig. 3 for an example of a spectrum of violet fluorescent light and Fig. 4 for an example of a spectrum of an LED). The number of light sources 10 may be one or may be plural depending on factors such as an intended intensity of irradiation and an intended range of irradiation of the light source.

The controller 20 is connected to the light source 10 by a wired connection or a wireless connection, and is configured to control irradiation conditions of the light source 10. The irradiation conditions can include at least one of the blinking frequency and the irradiation time of the light source 10, and therefore, the controller 20 can include at least one of a blinking frequency controller 20a and an irradiation time controller 20b. The blinking frequency may be preferably 0 Hz or a frequency in a range of 30 Hz to 75 Hz, and more preferably 0 Hz or a frequency in a range of 35 Hz to 45 Hz, and even more preferably 0 Hz or 40 Hz specifically. The blinking frequency of 0 Hz refers to constantly irradiated light. The irradiation time can be set arbitrarily to be in a range of 10 seconds to 24 hours per day for example, and a specific time for which the irradiation is to be continued can be set arbitrarily for a period of one day to few years, or more than that for example.

The controller 20 can include a processor such as a CPU (Central Processing Unit), and executes a processing of controlling irradiation conditions of the light source 10. The processing carried out by the controller 20 may be realized by a computer program or may be realized by hardware by a logic circuit. The computer program may be stored in a computer-readable recording medium. The recording medium having the computer program recorded may be a non-transient recording medium. The non-transient recording medium is not restricted in particular, and may be a recording medium such as a memory card and a CD-ROM (Compact Disc - Read Only Memory). The computer program stored in the recording medium can be installed in a computer unit via an appropriate reader. Examples of appropriate reader include a card reader in a case in which the recording medium is a memory card and a CD (Compact Disc) drive in a case in which the recording medium is a CD-ROM. Moreover, computer program may be a computer program downloaded to a computer unit via a communication network from an external server.

In the apparatus according to the present disclosure, the driving circuit may include at least one processor which is communicably connected to the light source and at least one memory, and at least one memory for storing commands that are executable by the processor.

The light source 10 of the apparatus for controlling biological function shown in Fig. 5 may be spectacles with a light source or a spectacle frame with a light source, a desktop light source, a mobile terminal mounted light source, a light source installed nearby or in front of face, a portable light source, an indoor lighting, or a table stand. Moreover, the apparatus for controlling biological function may be provided as a product with a light source having at least the light source 10 mounted thereon, out of the light source 10 and the controller 20, including spectacles or a spectacle frame, a desktop light, a mobile terminal, a case for a mobile terminal, a head-worn article (such as cap, earphone headphone), a portable light, an indoor lighting, or a table stand.

As described heretofore, the apparatus for controlling biological function by light stimulation according to the present disclosure, by irradiating light of a specific wavelength such as violet light constantly or at a specific blinking frequency to a living body, can lead to treatment and/or prophylaxis of Parkinson's disease, and to treatment and/or prophylaxis of at least one symptom selected from a group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis.

Moreover, one aspect of the present invention is related to a method of operating the apparatus for controlling biological function by light stimulation. Therefore, some embodiments of the present disclosure are related to a method of operating the apparatus which is used for controlling the abovementioned biological function, or in other words, for treatment and/or prophylaxis of Parkinson's disease, and treatment and/or prophylaxis of at least one symptom selected from a group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis, and the apparatus includes a light source which irradiates light of a specific wavelength constantly or at a specific blinking frequency to a living body and a controller which controls the blinking frequency of the light source, and the apparatus irradiates light of a specific wavelength constantly or at a specific blinking frequency to a living body. Here, the blinking frequency of the light source may be controlled to be 0 Hz or a frequency in a range of 30 Hz to 75 Hz, and moreover, the light source may be a light source which irradiates light of a wavelength in a range of 360 nm to 400 nm to a living body. In addition, one aspect of the present invention is related to a computer program which causes an apparatus including a light source which irradiates light of a specific frequency constantly or at a specific blinking frequency to a living body, and a controller which controls the blinking frequency of the light source, to execute the method of operating.

The computer program according to the present disclosure may have a command stored in a non-transitory computer-readable medium. The computer program according to the present disclosure can execute predetermined steps when the command is executed by the processor. Therefore, one aspect of the present disclosure is related also to a computer-readable medium which is a non-transitory computer-readable medium having commands stored therein, and as the command is executed by a processor, it is capable of executing for an apparatus including a light source which irradiates light of a specific wavelength constantly or at a specific blinking frequency to a living body, and a controller which controls a blinking frequency of the light source, a step of operating the apparatus to control the blinking frequency of the light source to be 0 Hz or to be a frequency in a range of 30 Hz to 75 Hz by the controller, and a step of operating the apparatus such that the light source irradiates light of a wavelength in a range of 360 nm to 400 nm to a living body.

Furthermore, one aspect of the present invention is related to an instrument for treatment and/or prophylaxis of Parkinson's disease by light stimulation, and the instrument may include a glass, a spectacle lens, or a contact lens which allows violet light to pass through. By using such instrument, it is possible to have a favorable effect on a living body such an improvement or suppression of at least one symptom selected from a group of symptoms consisting of cognitive memory disorder, working memory disorder, dysphoria, anxiety, apathy, psychological symptoms (hallucination, visual hallucination, and auditory hallucination), impulse control disorder, orthostatic hypotension, urinary disorder, sexual dysfunction, digestive symptoms (drooling, swallowing, nausea, constipation), sleep disorder, pain, weight loss, olfactory abnormality, fatigue, and diaphoresis. By using such instrument, treatment and/or prophylaxis of Parkinson's disease may be possible.

### Examples

### Example 1. Suppression of α-synuclein deposition by VL irradiation

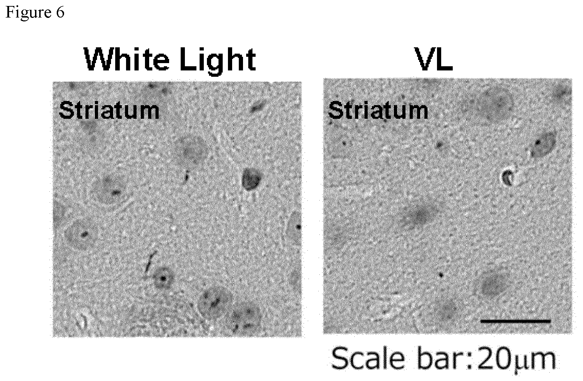

From one week before administering α-synuclein to the brain of mice, VL was irradiated (40 Hz; irradiation intensity of light incident on eyes 310 µW/cm²) for three hours per day, and moreover, after administering α-synuclein, VL was irradiated (irradiation intensity of light incident on eyes 310 µW/cm²) for three hours per day after two weeks up to six months, and sampling was done. As a result, it was observed that aggregate formation in striatum (detected by anti-phosphorylated synuclein (psyn) was delayed because of VL irradiation (Fig. 6). This result indicates the usability of violet light in Parkinson's disease which is a neurogenerative disorder. In other words, this indicates that it is possible to treat and/or prohibit Parkinson's disease by irradiating violet light.

### Example 2. Suppression of p62 protein deposition by VL irradiation

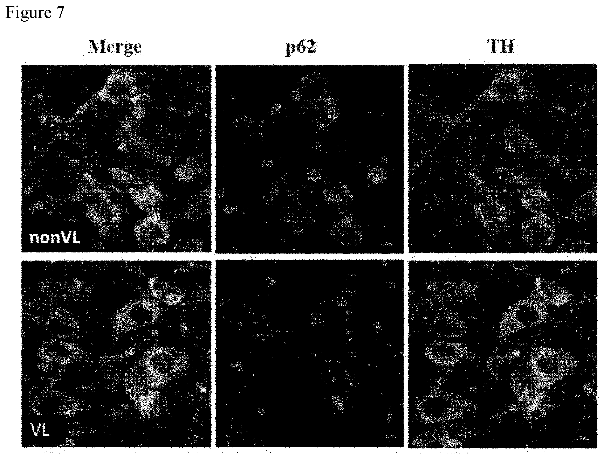

When violet light (40 Hz; irradiation intensity of light incident on eyes 310 µW/cm²) was irradiated for three hours per day during a period from young age up to old age to parkinsonian model mice (ATG7 KO mice; Japanese Unexamined Patent Application Publication No. 2018-068136) in which aggregates are formed due to defect of autophagy, an effect of aggregate suppression was observed (Fig. 7). This result indicates the usability of violet light in Parkinson's disease which is a neurogenerative disorder. In other words, this indicates that it is possible to treat and/or prevent Parkinson's disease by irradiating violet light.

### Example 3. Clinical test of patient suffering from Parkinson's disease

An effect of treatment of violet light on a patient suffering from Parkinson's disease which is a human was demonstrated by the following clinical test.

In a single group study for examples of 20 patients suffering from Parkinson's disease associated with visual hallucination in progress, the subjects wore goggles having violet light (irradiation intensity of light incident on eyes 310 µW/cm²) blinking at 40 Hz for three hours every day, for 12 weeks. Doctor in charge of research made safety evaluation by evaluating ophthalmic examination, adverse event, and problem occurrence status, and other evaluation scales, pareidolia test, MoCA-J, MDS-UPDRS parts I ~ IV (Movement Disorder Society - Unified Parkinson's Disease Rating Scale part III), MMSE (Mini Mental State Examination), PDQ (Parkinson's Disease Questionnaire) 39, PDSS-2 (Parkinson's Disease sleep scale - 2), Cognitive fluctuation inventory, NPI-Q (neuropsychiatric inventory questionnaire) were obtained.

By the abovementioned examination, it is possible to determine whether or not there is an effect of improvement in cognitive memory, working memory, QOL of helper, sleep, hallucination, visual hallucination, and auditory hallucination. Moreover, as the ophthalmic examination, visual acuity, intraocular pressure, slit-lamp microscopy, examination of the ocular fundus, retinal tomography, and optical coherence tomography angiography were evaluated.

The treatment and/or prophylaxis of Parkinson's disease by light stimulation was carried out according to an appropriate protocol based on the information provided in the present specification.

### Example 3-1, Case report of patients suffering from Parkinson's disease

When violet light was irradiated at 40 Hz for three hours during day time for 12 weeks to a 71-year-old patient of Parkinson's disease (irradiation intensity of light incident on eyes 310 µW/cm²), symptoms of sleep disorder and visual hallucination were alleviated. Furthermore, reoccurrence of visual hallucination and delirium was observed after the end of the irradiation period of violet light. This indicates that it is possible to treat and/or prevent Parkinson's disease by irradiating violet light.

### Example 4. Clinical test of patient suffering from Parkinson's disease (2)

An effect of treatment of violet light on a patient suffering from Parkinson's disease which is a human is demonstrated by the following clinical test.

In a test for a patient suffering from Parkinson's disease, the subject wears goggles having violet light (irradiation intensity of light incident on eyes 310 µW/cm²) irradiated constantly every day during the day time. An effect of treatment on the subject is evaluated similarly as in Example 3.

All publications, applications, standards, and patents mentioned in present specification are herein incorporated by reference in its entirety, and in a case of contradictory terminology, the present specification will take precedence. Scope of the present invention is not restricted by specific embodiments described in the present specification. In fact, in addition to the description in the present specification, various modifications of the present invention will be apparent to the person skilled in the art from the abovementioned description and the accompanying drawings. Such amendments are intended to fall within the scope of the appended patent claims. Some or all of the embodiments may also be described as the following supplementary notes, and the disclosure of the present application is not restricted to the following supplementary notes.

## Claims

1. A method for treatment and/or prophylaxis of Parkinson's disease, comprising:
irradiating light of a specific wavelength to a subject by using a light irradiating apparatus.

2. The method according to claim 1, wherein the method for treatment and/or prophylaxis of Parkinson's disease is a method for treatment and/or prophylaxis of motor symptoms and/or non-motor symptoms of Parkinson's disease

3. The method according to claim 2, wherein the motor symptom is at least one symptom selected from a group of symptoms consisting of, difficulty in speaking, ptyalism (excessive drooling), difficulty in chewing, difficulty in swallowing, difficulty in feeding action, difficulty in changing clothes, difficulty in maintaining personal hygiene, difficulty in writing, decrease in hobbies and entertainment, difficulty in turning over, tremor, difficulty in getting up from bed, chair, and wheelchair, difficulty in walking, loss of balance, freezing of gait (FOG), stiffness in walking, difficulty in speech, loss of facial expression, rigidity, finger tapping, impairment in hand movement, impairment in pronation and supination movement of the hand, toe tapping, impairment in agility of the lower extremities, impairment in postural stability, difficulty in maintaining posture, general spontaneity of movement (bradykinesia), hand posture tremor, hand movement tremor, resting tremor amplitude, resting tremor, and restless leg syndrome.

4. The method according to one of claims 2 and 3, wherein the non-motor symptom is at least one symptom selected from a group of symptoms consisting of cognitive disorder, attentional function disorder, cognitive memory disorder, working memory disorder, depressed mood, feeling of anxiety, feeling of urgency, apathy, psychological symptoms (hallucination, visual hallucination, delusion, and auditory hallucination), dopamine dysregulation syndrome, impulse control disorder, orthostatic hypotension, postural hypotension, urinary disorder, sexual dysfunction, digestive symptoms (drooling, swallowing, nausea, constipation), sleep disorder, pain and other paresthesia (olfactory abnormality and visual abnormality), weight change, fatigue, diaphoresis, and syncope.

5. The method according to claim 4, wherein at least one symptom selected from the group of symptoms consisting of cognitive disorder, attentional function disorder, cognitive memory disorder, working memory disorder, depressed mood, feeling of anxiety, feeling of urgency, apathy, psychological symptoms (hallucination, visual hallucination, and auditory hallucination), dopamine dysregulation syndrome, impulse control disorder, orthostatic hypotension, postural hypotension, urinary disorder, sexual dysfunction, digestive symptoms (drooling, swallowing, nausea, constipation), sleep disorder, pain and other paresthesia (olfactory abnormality and visual abnormality), weight change, fatigue, diaphoresis, and syncope is attributable to Parkinsons's disease.

6. The method according to any one of claims 1 to 5, wherein the method for treatment and/or prophylaxis of Parkinson's disease is a method for treatment and/or prophylaxis of dyskinesia.

7. The method according to any one of claims 1 to 6, wherein Parkinson's disease is **characterized by** being classified according to Hoehn & Yahr severity level classification, as one of level I, level II, level III, level IV, or level V.

8. The method according to any one of claims 1 to 7, wherein the light of a specific wavelength is irradiated constantly or at a specific blinking frequency to the subject.

9. The method according to any one of claims 1 to 8, wherein the subject is being administered or was administered at least one medical agent selected from a group consisting of levodopa, phenylalanine, tyrosine, bromocriptine mesylate, pergolide mesylate, cabergoline, talipexole hydrochloride, pramipexole hydrochloride, ropinirole hydrochloride, rotigotine, apomorphine hydrochloride hydrate, trihexyphenidyl hydrochloride, biperiden, selegiline, safinamide, rasagiline, entacapone, opicapone, droxidopa, istradefylline, quetiapine, zonisamide, and amantadine.

10. The method according to any one of claims 1 to 9, wherein the light is violet light.

11. The method according to any one of claims 1 to 10, wherein the specific wavelength includes a wavelength in a range of 360 nm to 400 nm.

12. The method according to any one of claims 1 to 11, wherein the specific wavelength includes a wavelength of approximately 380 nm.

13. The method according to any one of claims 1 to 12, wherein the light is one of light irradiated constantly and light blinking at a specific blinking frequency.

14. The method according to claim 13, wherein the blinking frequency is in a range of 30 Hz to 70 Hz.

15. The method according to one of claims 13 and 14, wherein the blinking frequency is in a range of 35 Hz to 60 Hz.

16. The method according to any one of claims 13 to 15, wherein the blinking frequency is 40 Hz.

17. The method according to any one of claims 1 to 16, wherein the light is irradiated during daytime.

18. The method according to any one of claims 1 to 17, wherein the light is irradiated for 10 seconds or more, as a total time of light irradiation.

19. The method according to any one of claims 1 to 18, wherein the light is irradiated such that irradiance of light incident on eyes is within a range of 0.5 µW/cm² to 1000 µW/cm².

20. The method according to any one of claims 1 to 19, wherein the light is irradiated for five minutes or more, as a total time of light irradiation.

21. The method according to any one of claims 1 to 20, wherein the light irradiating apparatus is spectacles with a light source or a spectacle frame with a light source, a desktop light source, a mobile terminal mounted light source, a light source installed nearby or in front of face, a portable light source, an indoor lighting, and a desk lamp.

22. The method according to any one of claims 1 to 21, wherein the subject is a human.

23. An apparatus for treatment and/or prophylaxis of Parkinson's disease by irradiating light of a specific wavelength constantly or at a specific blinking frequency to a subject, comprising:
a light source which irradiates light of a specific wavelength constantly or at a specific blinking frequency.

24. The apparatus according to claim 23, further comprising:
a controller which controls emission of light which induces specific brain waves same as, or substantially same as, or different from an irradiation state of light, in the subject to which the light is irradiated.

25. The apparatus according to claim 24, wherein the controller, by transceiving to and from an isolated controller such as a portable terminal, carries out control by changing irradiation conditions such as the blinking frequency of light of a specific wavelength, irradiance, irradiation time, irradiation-start time, and irradiation-end time.

26. The apparatus according to claim 25, further comprising:
a driving circuit which drives the light source.

27. The apparatus according to claim 26,
wherein the driving circuit includes at least one processor and at least one memory for storing commands that are executable by the processor, and
the processor is communicably connected to the light source and the memory.

28. The apparatus according to any one of claims 23 to 27, wherein the apparatus for treatment and/or prophylaxis of Parkinson's disease treats and/or prevents motor symptoms and/or non-motor symptoms of Parkinson's disease.

29. The apparatus according to claim 28, wherein the motor symptom is at least one symptom selected from a group of symptoms consisting of difficulty in speaking, ptyalism (excessive drooling), difficulty in chewing, difficulty in swallowing, difficulty in feeding action, difficulty in changing clothes, difficulty in maintaining personal hygiene, difficulty in writing, decrease in hobbies and entertainment, difficulty in turning over, tremor, difficulty in getting up from bed, chair, and wheelchair, difficulty in walking, loss of balance, freezing of gait (FOG), stiffness in walking, difficulty in speech, loss of facial expression, rigidity, finger tapping, impairment in hand movement, impairment in pronation and supination movement of hand, toe tapping, impairment in agility of the lower extremities, impairment in postural stability, difficulty in maintaining posture, general spontaneity of movement (bradykinesia), hand posture tremor, hand movement tremor, resting tremor amplitude, resting tremor, and restless leg syndrome.

30. The apparatus according to one of claims 28 and 29, wherein the non-motor symptom is at least one symptom selected from a group of symptoms consisting of cognitive disorder, attentional function disorder, cognitive memory disorder, working memory disorder, depressed mood, feeling of anxiety, feeling of urgency, apathy, psychological symptoms (hallucination, visual hallucination, delusion, and auditory hallucination), dopamine dysregulation syndrome, impulse control disorder, orthostatic hypotension, postural hypotension, urinary disorder, sexual dysfunction, digestive symptoms (drooling, swallowing, nausea, constipation), sleep disorder, pain and other paresthesia (olfactory abnormality and visual abnormality), weight change, fatigue, diaphoresis, and syncope.

31. The apparatus according to claim 30, wherein at least one symptom selected from a group of symptoms consisting of cognitive disorder, attentional function disorder, cognitive memory disorder, working memory disorder, depressed mood, feeling of anxiety, apathy, psychological symptoms (hallucination, visual hallucination, delusion, and auditory hallucination), dopamine dysregulation syndrome, impulse control disorder, orthostatic hypotension, postural hypotension, urinary disorder, sexual dysfunction, digestive symptoms (drooling, swallowing, nausea, constipation), sleep disorder, pain and other paresthesia (olfactory abnormality and visual abnormality), weight change, fatigue, diaphoresis, and syncope is attributable to Parkinson's disease.

32. The apparatus according to any one of claims 23 to 31, wherein the apparatus for treatment and/or prophylaxis of Parkinson's disease treats and/or prevents dyskinesia.

33. The apparatus according to any one of claims 23 to 32, wherein Parkinson's disease, according to Hoehn & Yahr severity level classification, is classified as one of level I, level II, level III, level IV, and level V.

34. The apparatus according to any one of claims 23 to 33, wherein the subject is being administered or was administered at least one medical agent selected from a group consisting of levodopa, phenylalanine, tyrosine, bromocriptine mesylate, pergolide mesylate, cabergoline, talipexole hydrochloride, pramipexole hydrochloride ropinirole hydrochloride, rotigotine, apomorphine hydrochloride hydrate, trihexyphenidyl hydrochloride, biperiden, selegiline, safinamide, rasagiline, entacapone, opicapone, droxidopa, istradefylline, quetiapine, zonisamide, and amantadine.

35. The apparatus according to any one of claims 23 to 34, wherein the light is violet light.

36. The apparatus according to any one of claims 23 to 35, wherein the specific wavelength includes a wavelength in a range of 360 nm to 400 nm.

37. The apparatus according to any one of claims 23 to 36, wherein the specific wavelength includes a wavelength of approximately 380 nm.

38. The apparatus according to any one of claims 23 to 37, wherein the blinking frequency is in a range of 30 Hz to 70 Hz.

39. The apparatus according to any one of claims 23 to 38, wherein the blinking frequency is in a range of 35 Hz to 60 Hz.

40. The apparatus according to any one of claims 23 to 39, wherein the blinking frequency is 40 Hz.

41. The apparatus according to any one of claims 23 to 40, wherein the light is irradiated during daytime.

42. The apparatus according to any one of claims 23 to 41, wherein the light is irradiated for 10 seconds or more, as a total time of light irradiation.

43. The apparatus according to any one of claims 23 to 42, wherein the light is irradiated such that irradiance of light incident on eyes is within a range of 0.5 µW/cm² to 1000 µW/cm².

44. The apparatus according to any one of claims 23 to 43, wherein the light is irradiated for five minutes or more, as a total time of light irradiation.

45. The apparatus according to any one of claims 23 to 44, wherein the apparatus is spectacles with a light source or a spectacle frame with a light source, a desktop light source, a mobile terminal mounted light source, a light source installed nearby or in front of face, a portable light source, an indoor lighting, and a desk lamp.

46. The apparatus according to any one of claims 23 to 45, wherein light of different wavelength, sound, vibrations, magnetic field, and electric field are applied together with the irradiation of light of a specific wavelength.

47. computer-readable medium which is a non-transitory computer-readable medium having commands stored therein, and which is capable of causing an apparatus according to any one of claims 23 to 46 including a light source which irradiates light of a specific wavelength constantly or at a specific blinking frequency to a living body and a controller which controls the blinking frequency of the light source, to execute the following steps when a command is executed by a processor:
a step of operating the apparatus such that the controller controls the blinking frequency of the light source to 0 Hz or to a frequency in a range of 30 Hz to 75 Hz; and
a step of operating the apparatus such that the light source irradiates light of a wavelength in a range of 360 nm to 400 nm to a living body.

48. An instrument for treatment and/or prophylaxis of Parkinson's disease by light stimulation, comprising:
a glass, a spectacle lens, or a contact lens which allows violet light to pass through.
